(19) Europäisches Patentamt
European Patent Office
Office européen des brevets

(11) Publication number: **0 052 964**
**A1**

(12) # EUROPEAN PATENT APPLICATION

(21) Application number: **81305218.0**

(22) Date of filing: **03.11.81**

(51) Int. Cl.³: **C 07 D 473/34**
A 61 K 31/52

(30) Priority: **20.11.80 GB 8037298**

(43) Date of publication of application:
**02.06.82 Bulletin 82/22**

(84) Designated Contracting States:
**BE CH DE FR IT LI NL**

(71) Applicant: **BEECHAM GROUP PLC**
**Beecham House Great West Road**
**Brentford Middlesex(GB)**

(72) Inventor: **Ferres, Harry**
**20 Garlichill Road**
**Epsom Surrey(GB)**

(72) Inventor: **Tyrrell, Arthur William**
**20 Sandhills Road**
**Reigate Surrey(GB)**

(72) Inventor: **Geen, Graham Richard**
**32 Queensway North**
**Walton on Thames Surrey(GB)**

(74) Representative: **Cresswell, Thomas Anthony et al,**
**European Patent Attorney Beecham Pharmaceuticals**
**Great Burgh Yew Tree Bottom Road**
**Epsom Surrey KT18 5XQ(GB)**

(54) **Adenine derivatives possessing pharmacological activity.**

(57) Compounds of formulae (I) and (II) and salts thereof

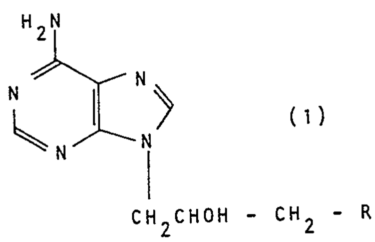

(1)

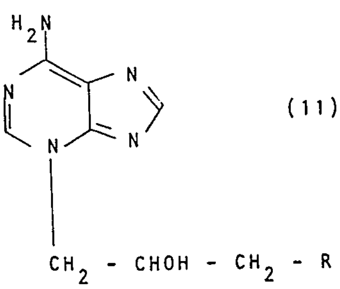

(11)

have hypolipidaemic activity.

EP 0 052 964 A1

0052964

# Adenine Derivatives possessing Pharmacological Activity

The present invention relates to derivatives of adenine having hypolipidaemic activity, to processes for their preparation and to their use in medicine.

The present invention provides the compounds of the formulae (I) and (II) and salts thereof:

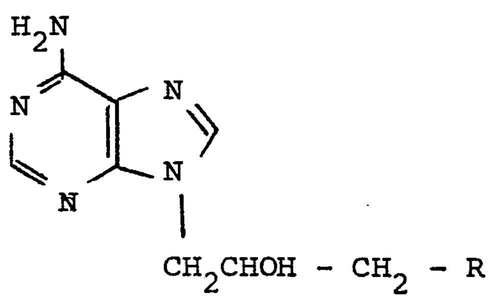

(I)

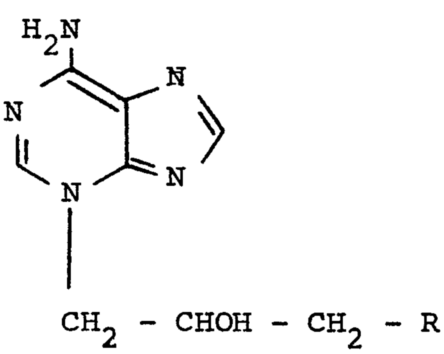

(II)

wherein R is an optionally substituted group of formula (III):

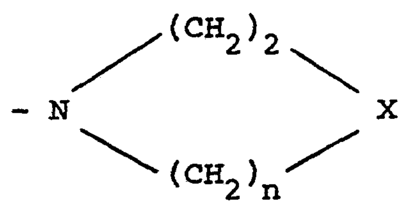

(III)

wherein X is an oxygen or sulphur atom; and
n is 1, 2 or 3.

any substituents being selected from $C_{1-4}$ alkyl groups,
$C_{1-4}$ polymethylene groups attached to two different atoms thus forming a bicyclic ring system, and
$C_{2-5}$ polymethylene groups attached to one carbon atom thus forming a spirocycloalkyl group,

provided that R is other than an unsubstituted morpholine group.

Suitable examples of R include substituted morpholino and substituted or unsubstituted thiomorpholino groups.

Preferably n is 2.

Suitable example of $C_{1-4}$ alkyl groups include methyl, ethyl, *n*- and *iso*-propyl and *n*-butyl. Methyl groups are preferred. When two or more alkyl substituents are present they are preferably the same.

When two or more alkyl substituents are present they may be on the same or different carbon atoms.

Suitable examples of a $C_{1-4}$ polymethylene bridging group include ethan-1,2-diyl and propan-1,3-diyl. When a bridging group is present, preferably the bridgehead carbon atoms are unsubstituted.

Suitable examples of a $C_{2-5}$ polymethylene spirocycloalkyl group include spirocyclopentyl.

When a polymethylene bridging or spirocycloalkyl group is present, it is preferred that there are no other substituents present.

Compounds of formulae (I) and (II) have an asymmetric carbon atom (ie that bearing the hydroxyl group) and may thus exist in at least two stereoisomeric forms. The group R may also include one or more asymmetric carbon atoms.

The present invention encompasses all stereoisomeric forms of the compounds of formulae (I) and (II) whether free from other stereoisomers or admixed with other stereoisomers. It, therefore, includes optically pure enantiomers and racemic mixtures thereof.

The different stereoisomeric forms may be separated by conventional methods or produced by stereospecific or asymmetric synthesis.

The compounds of this invention may be provided in the form of solvates such as hydrates.

The salts of the compounds of formulae(I) and (II) include acid addition salts and these are preferably acid addition salts with pharmaceutically acceptable acids. Such acids may be inorganic or organic acids such as

hydrochloric, hydrobromic, orthophosphoric, sulphuric, methanesulphonic, acetic, citric, lactic, tartaric, propionic, benzoic and fumaric acids.

The salts of the compounds of the formulae (I) and (II) also include pharmaceutically acceptable quaternary ammonium salts. Examples of such salts include such compounds quaternised by compounds such as $R^5$ - Y wherein $R^5$ is $C_{1-6}$ alkyl, phenyl - $C_{1-6}$ alkyl, $C_{5-7}$ cycloalkyl, or in vivo hydrolysable substituted $C_{1-4}$ alkyl groups and Y is an anion of an acid. Suitable examples of $R^5$ include methyl, ethyl and n- and iso-propyl; and benzyl and phenylethyl.

Examples of substituted $C_{1-4}$ alkyl groups which hydrolyse readily in the body to produce the parent amine include acyloxyalkyl groups such as acetoxymethyl, pivaloyloxymethyl, α-acetoxyethyl, α-acetoxybenzyl and α-pivaloyloxyethyl groups; and alkoxycarbonyloxyalkyl groups, such as ethoxycarbonyloxymethyl and α-ethoxycarbonyloxyethyl. Suitable examples of Y include the halides such as chloride, bromide and iodide.

Crystalline acid addition salts are particularly apt.

It will be appreciated that one group of compounds of formulae (I) and (II) which are of particular interest are those wherein R is a substituted or unsubstituted monocyclic group. A further group of compounds of particular interest are those wherein R is an unsubstituted bicyclic group.

Examples of compounds of formula (I) include:

6-amino-9-[(2-hydroxy-3-thiomorpholino)propyl]purine, and
6-amino-9-([2-hydroxy-3-(2,6-cis-dimethylmorpholino)]propyl)purine.

Examples of compounds of formula (II) include:

6-amino-3-[(2-hydroxy-3-thiomorpholino)propyl]-purine, and
6-amino-3-([2-hydroxy-3-(2,6-cis-dimethylmorpholino)] propyl)-purine.

The present invention also provides a process for the preparation of a compound of the formula (I) or (II) as hereinbefore defined, which process comprises:

a. the reaction of adenine or a metal salt thereof with a compound of the formula (VIII):

$$Z - CH_2 - CHA - CH_2 - R \qquad (VIII)$$

wherein: R is as defined in formulae (I) and (II); and A is hydroxyl and Z is a group readily displaceable by a nucleophile; or A and Z together form an oxide diradical; or

b. the reaction of a compound of the formula (IX) or (X):

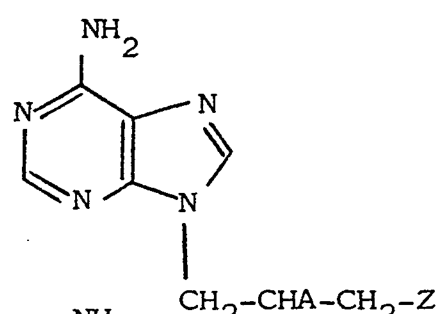

(IX)

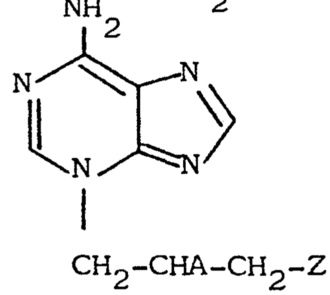

(X)

wherein: A and Z are as hereinbefore defined, with a compound of the formula (XI):

H - R (XI)

wherein: R is as defined in formulae (I) and (II); or

c. the reduction of a compound of the formula (XII) or (XIII):

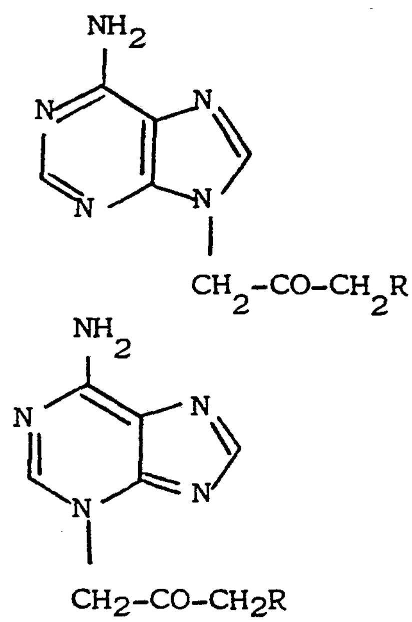

(XII)

(XIII)

wherein R is as defined in formulae (I) and (II); or

d. the deprotection of an N- or O- protected compound corresponding to a compound of the formula (I) or (II); or

e. for a compound of the formula (I), the reaction of formamide with a compound of the formula (XIV):

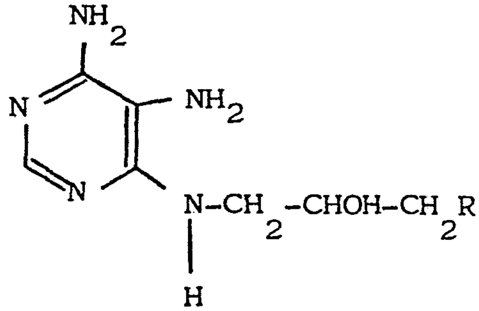

(XIV)

wherein R is as defined in formulae (I) and (II); and optionally thereafter salifying the compound of the formula (I) or (II) so produced.

Process variants a and d are preferred processes of the invention.

In process variant a suitable examples of Z when a group readily displaceable by a nucleophile include halogen atoms such as bromine or chlorine, and activated ester residues such as methanesulphonyloxy or toluenesulphonyloxy.

If adenine _per se_ is employed in process variant a then an inorganic base, for example an alkali metal base such as the hydroxide or carbonate, for example potassium hydroxide or carbonate, is used to form a metal salt _in situ_; alternatively, a pre-formed metal salt, for example the potassium salt, is used.

The reaction is usually carried out in a lower alkanol such as ethanol or isopropanol at an elevated temperature such as at reflux. The crude product may be obtained by evaporation of the filtered reaction mixture. This initial product may be purified by conventional methods such as re-crystallisation.

The reaction of process variant a results in the preparation of compounds of the formulae (I) and (II) (c.f. J.H. Lister, "The Chemistry of Heterocyclic Compounds; Fused Pyrimidines, Part II (Purines)" (Wiley-Interscience, 1971), and this process thus also comprises the optional separation of the compounds of formulae (I) and (II) by conventional means. If both salification and separation are carried out, then they may be carried out in any appropriate order.

Separation of the compounds of the formulae (I) and (II) may be effected by column chromatography, for example over silica using a solvent such as chloroform or ethyl acetate optionally in the presence of a lower alkanol such as methanol.

It will be apparent that optional salification appropriately follows separation in such a case.

Reaction conditions for process variant b are substantially those for variant a. The presence of base is optional, but preferred when Z is halogen. Excess of the compound of formula (XI) may be used as an alternative base.

For process variant c, suitable examples of reducing agents include borohydrides, such as sodium or lithium borohydrides, which may be used in a solvent such as diglyme, a lower alcohol or an ether, at non-extreme temperatures, such as ambient temperature.

Process variant e is suitably carried out analogously to the methods of Bredereck et al., given in Chem. Ber., 86, 333, (1953) and U.K. Patent No. 706,424.

Process variant d is preferred for the preparation of compounds of the formula (I), in particular when the process comprises the removal of the oxide radical from a compound of the formula (XV):

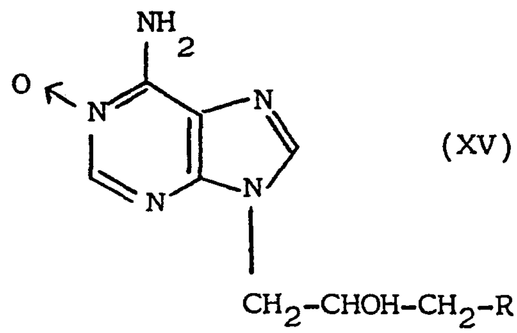

(XV)

The reaction may be carried out by catalytic hydrogenolysis, for example in the presence of Raney nickel. Such catalytic hydrogenolysis may be carried out with hydrogen at or in slight excess of atmospheric pressure at non-extreme temperatures, for example ambient temperature, in an inert polar solvent, such as a lower alkanol, for example ethanol.

Compounds of the formula (XV) may be prepared by the reaction of adenine-1-oxide with a compound of the formula (VIII) as hereinbefore defined, under the reaction conditions described for process variant a.

When a compound of the formula (I) or (II) is capable of existing as a number of diastereomers as discussed hereinbefore, a mixture of isomers of the compound of the

formula (I) or (II) may be synthesised non-stereospecifically and the desired isomer separated conventionally therefrom, e.g. by chromatography; or alternatively the pure isomer may if desired be synthesised from the corresponding isomeric form of the relevant starting material, e.g. compounds of the formulae (VIII) or (XI).

Isomeric forms of the compounds of the formulae (VIII) or (XI) are either known as separate forms or may be separated or resolved conventionally e.g. by chromatography.

Racemates of compounds of the formulae (I) or (II) or (VIII) or (XI) may be resolved conventionally, e.g. by salification with a chiral acid and separation of the resultant salts, for example as in L.F. Fieser and M. Fieser, 'Organic Chemistry', 3rd Ed., 1956, Reinhold; S.H. Wilen 'Tables of Resolving Agents and Optical Resolutions', University of Notre Dame Press, 1972, or S.H. Wilen, 'Topics in Stereochemistry', 1971, Vol. 6, John Wiley, NY.

The free bases of the compounds of the formulae (I) and (II) may be salified by treatment with an acid or treatment with a compound $R^5Y$ as hereinbefore defined.

In general, within acid addition salts, di-salts are preferred, since they are more easily prepared using a slight excess of the relevant acid.

It will also be realised that salts of the compounds of the formulae (I) and (II) which are not pharmaceutically acceptable may be useful as intermediates in the preparation of pharmaceutically acceptable salts of compounds of the formulae (I) and (II) or the compounds of the formulae (I) and (II) themselves, and as such form an aspect of the present invention.

The present invention also provides a pharmaceutical

composition which comprises a compound of this invention as hereinbefore described together with a pharmaceutically acceptable carrier therefore. Preferred compositions are in a form suitable for oral administration. Oral dosage forms may contain such conventional excipients such as fillers, lubricants, disintegrants, binders, preservatives, colourants and so on. Suitable fillers include lactose, microcrystalline cellulose, calcium phosphate, mannitol and the like. Suitable lubricants include magnesium stearate and stearic acid. Suitable disintegrants include polyvinylpolypyrrolidone and sodium starch glycollate.

The oral dosage forms are normally provided as discrete unit forms such as tablets and capsules. In general such unit dosage forms will contain from 5 to 500 mgs and more usually from 25 to 300 mgs. These unit dosage forms may be administered form 1 to 6 times daily in such a way that the daily dose for a 70 kg adult will normally be between 30 to 1500 mgs and more usually from 100 to 1000 mgs, for example from 200 to 800 mgs.

The present invention further provides a method of treating hyperlipidaemia in humans which method comprises administering an effective, non-toxic amount of a compound of formula (I) or (II) to a hyperlipidaemic human.

The following examples illustrate the preparation of the compounds of the invention and the preparation of intermediates therefore.

EXAMPLE 1

6-Amino-9-[(2-hydroxy-3-thiomorpholino)propyl]-purine (1)

and

6-Amino-3-[(2-hydroxy-3-thiomorpholino)propyl]-purine (2)

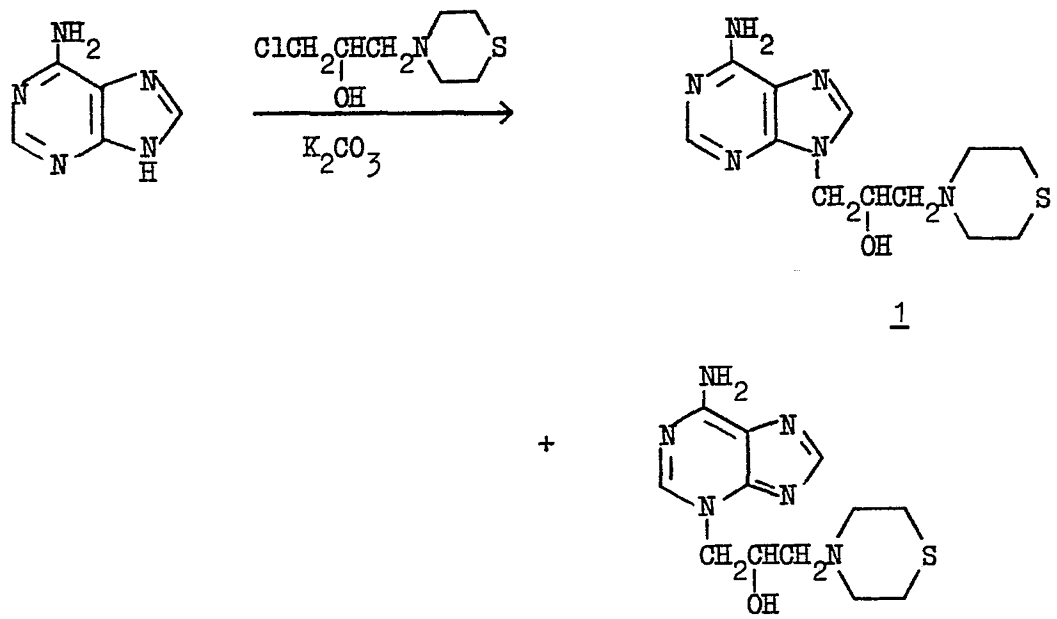


1

Thiomorpholine (10g., 0.1mole) was dissolved in propan-2-ol (50ml) and maintained under $N_2$ while epichlorohydrin (8.98g., 0.1mole) was added dropwise with stirring at room temperature over a period of 1 hr. The mixture was left to stand under $N_2$ overnight and the alkylating agent formed was used without purification as described below.

Adenine (6.75g., 0.05mole) was suspended in propan-2-ol (50ml), anhydrous potassium carbonate (10.35g., 0.075mole) was added, and the mixture stirred under reflux for 75 min. To the suspension was added, at reflux, 1-chloro-3- thiomorpholino -propan-2-ol (ca. 0.06mole) in propan-2-ol (40ml), prepared as described above. The mixture was stirred under reflux for 1 hr., filtered while hot, and the inorganic material was washed well with hot propan-2-ol. The filtrate was evaporated giving a gum which solidified when triturated with cold ethyl acetate. The mixture of products obtained (18.6g) was purified by column chromatography on silica gel (2 x 180g) using dichloromethane-methanol-aqueous ammonia (93.5:6:0.5) as eluant.

The product of higher $R_f$ was recrystallized from ethyl acetatemethanol (9:1) yielding 6-amino-9-[(2-hydroxy-3-thiomorpholino)propyl]-purine (3.65g., 25%) m.p. 179-180°. (Compound 1)

I.R. Spectrum (KBr disc): $\nu_{max}$ 3320, 3145, 1660, 1600, 1580 $cm^{-1}$.

N.M.R. Spectrum (DMSO-$d_6$; 80MHz): $\delta$ 8.15 (s, 1H) and 8.05 (s, 1H) (protons in purine nucleus); 7.13 (br s, 2H, $NH_2$); 5.04 (d, 1H, OH); 4.50-3.80 (m, 3H, $N_{(9)}CH_2CH$); 2.80-2.10 [m, 10H, $N(CH_2)_3$, $S(CH_2)_2$].

The more polar product was recrystallized from methanol giving 6-amino-3-[(2-hydroxy-3-thiomorpholino)propyl]-purine (2.0g., 14%) m.p. 231-235° (dec). (Compound 2).

I.R. Spectrum (KBr disc): $\nu_{max}$ 3220, 3100, 1715, 1640 $cm^{-1}$.

N.M.R. Spectrum (DMSO-$d_6$; 80MHz): $\delta$ 8.21 (s, 1H) and 7.75 (s, 1H) (protons in purine nucleus); 7.87 (br s, 2H, $NH_2$); 5.26 (br d, 1H, OH); 4.65-3.90 (m, 3H, $N_{(3)}CH_2CH$); 3.00-2.00 [m, 10H, $N(CH_2)_3$, $S(CH_2)_2$].

EXAMPLE 2

6-Amino-9-([2-hydroxy-3-(2,6-cis-dimethylmorpholino)]propyl)-purine (3)

and

6-Amino-3-([2-hydroxy-3-(2,6-cis-dimethylmorpholino)]propyl)-purine (4)

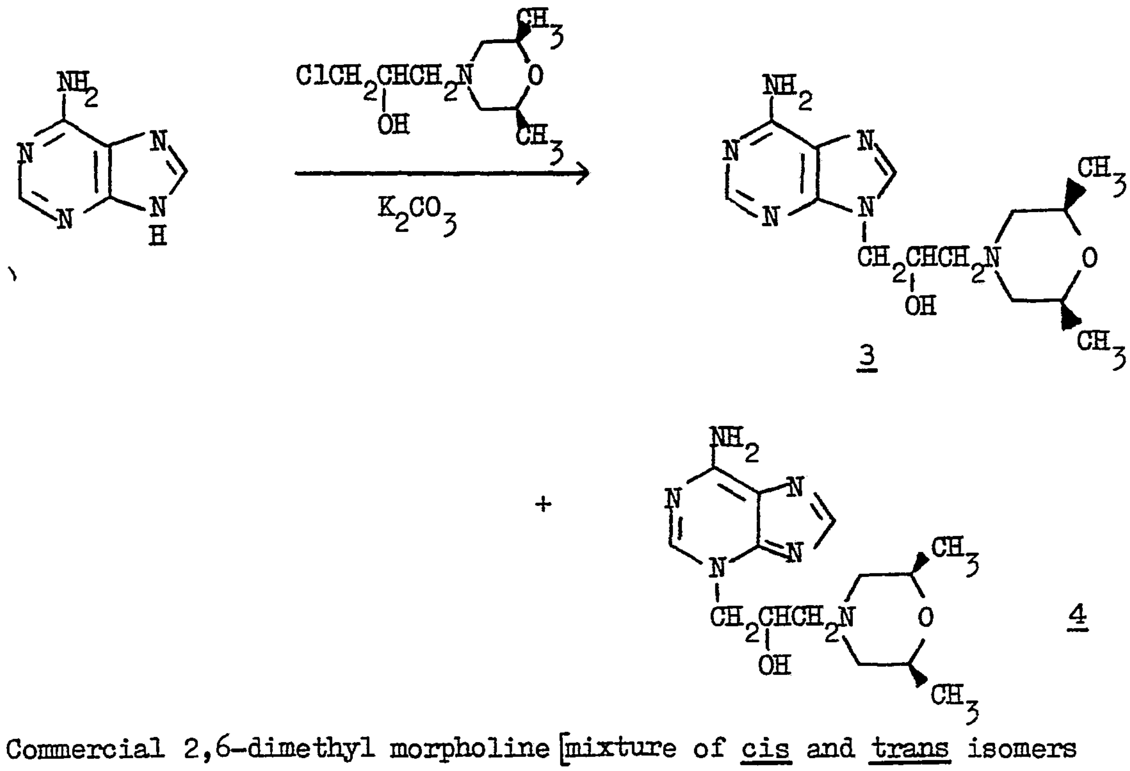


3

\+

4

Commercial 2,6-dimethyl morpholine [mixture of cis and trans isomers (6.9g., 0.06mole)] was dissolved in propan-2-ol (50ml), and epichlorohydrin (5.55g., 0.06mole) was added dropwise with stirring at room temperature over a period of 1 hr. The mixture was left to stand overnight and the alkylating agent formed was used without purification as described below.

Adenine (6.75g., 0.05mole) was suspended in propan-2-ol (50ml), anhydrous potassium carbonate (10.35g., 0.075mole) was added, and the mixture was stirred under reflux for 75 min. To the suspension was added, at reflux, 1-chloro-3-(2,6-dimethylmorpholino)propan-2-ol (0.06mole) in propan-2-ol (50ml), prepared as described above.

The mixture was stirred under reflux for 1 hr., filtered while hot, and the inorganic material was washed well with hot propan-2-ol. The filtrate was evaporated, giving a gum which partially solidified when

triturated with cold ethyl acetate. The crude solid was filtered off and the solid (7g) and filtrate were separately purified by column chromatography, each on silica gel (180g) using dichloromethane-methanol-aqueous ammonia as eluant (93.5:6:0.5 for the solid; 94.5:5:0.5 for the filtrate).

The product of second-highest $R_f$ was recrystallized from ethyl acetate/methanol (9:1) yielding 6-amino([2-hydroxy-3-(2,6-cis-dimethylmorpholino)]propyl)purine, (total yield from both columns was 4.88g., 32%), m.p. 121-124°. (Compound 3)

I.R. Spectrum (KBr disc): $\nu_{max}$ 3430 (sh), 3310, 3150, 1690. 1650, 1615, 1605. 1580 $cm^{-1}$.

N.M.R. Spectrum (DMSO-$d_6$; 80MHz): $\delta$ 8.15 (s, 1H) and 8.06 (s, 1H) (protons in purine nucleus); 7.17 (br s, 2H, $NH_2$); 5.06 (d, 1H, OH); 4.52-3.82 (m, 3H, $N_{(9)}CH_2CH$); 3.49 (br t, 2H, $(CH)_2O$); 2.87-2.12 (m, 4H, $NCH_2CHOH$, $\frac{1}{2}NCH_2CHCH_3$); 1.60 (t, 2H, $\frac{1}{2}NCH_2CHCH_3$); 0.99 (d, 6H, $(CH_3)_2$).

The most polar product was recrystallized from ethanol giving 6-amino-3-([2-hydroxy-3-(2,6-cis-dimethylmorpholino)]propyl)purine (0.4g., 3%), m.p. 224-225°. (Compound 4).

I.R. Spectrum (KBr disc): $\nu_{max}$ 3230, 3110, 1700, 1635 $cm^{-1}$.

N.M.R. Spectrum (DMSO-$d_6$; 80MHz): $\delta$ 8.23 (s, 1H) and 7.76 (s, 1H) (protons in purine nucleus); 7.89 (br s, 2H, $NH_2$); 5.26 (br s, 1H, OH); 4.75-3.85 (m, 3H, $N_{(3)}CH_2CH$); 3.75-3.20 (m, 2H, $(CH)_2O$); 3.00-3.20 (m, 4H, $NCH_2CHOH$, $\frac{1}{2}NCH_2CHCH_3$); 1.85-1.35 (m, 2H, $\frac{1}{2}NCH_2CHCH_3$); 1.00 (d, 6H, $(CH_3)_2$).

The corresponding trans isomers, ie 6-amino-9-([2-hydroxy-3-(2,6-trans-dimethylmorpholino)]propyl)purine and 6-amino-3-([2-hydroxy-3-(2,6-trans-dimethylmorpholino)]propyl)purine may also be isolated from the reaction mixture.

The hypocholesterolaemic and hypotriglyceridaemic effects of 6-amino-9-[(2-hydroxy-3-thiomorpholino)propyl]purine (Compound 1) and 6-amino-9-([2-hydroxy-3-(2,6-*cis*-dimethylmorpholino)]propyl)-purine (Compound 2) were assessed as follows.

Groups of 8 male rats (Sprague-Dawley from Tuck's) weighing 80-100g were adapted to a sucrose-rich semisynthetic diet containing 0.25% cholesterol by progressive dilution over 7 days of a powdered commercially available diet (Oxoid). Compounds were then added to the semisynthetic diet at the level of 0.1% and these diets were fed for 7 days. The rats were then killed and the serum total cholesterol and triglyceride were measured by a Technicon Auto-Analyser. The results are expressed as percentage change from control values.

| Compound | Serum Cholesterol | Serum Triglyceride | Liver wt. as % body wt. |
|---|---|---|---|
| 1 | -57* | -8 | +1 |
| 2 | -44* | -36* | +1 |

No drug induced deaths occur at the therapeutic dose.

* Indicates statistically significant results.

CLAIMS

1. A compound of formula (I) or (II) or a salt thereof:

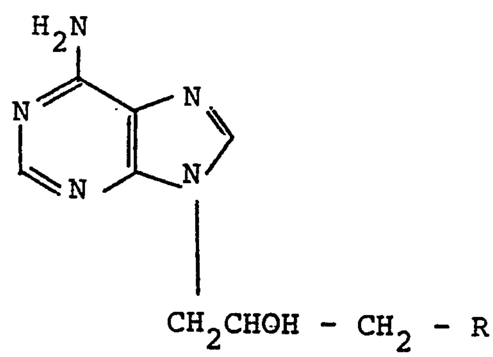

(I)

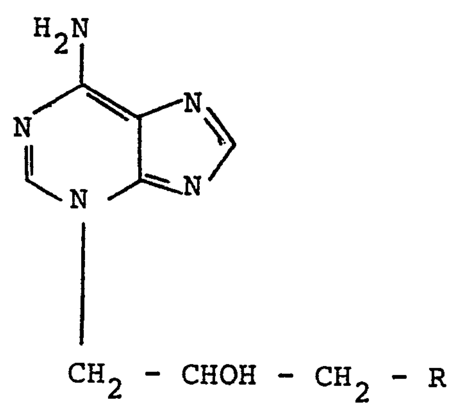

(II)

wherein R is an optionally substituted group of formula (III):

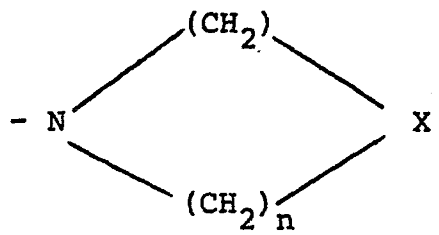

(III)

wherein X is an oxygen or sulphur atom; and
n is 1, 2 or 3.
any substituents being selected from
$C_{1-4}$ alkyl groups,
$C_{1-4}$ polymethylene groups attached to two different atoms thus forming a bicyclic ring system, and
$C_{2-5}$ polymethylene groups attached to one carbon atom thus forming a spirocycloalkyl group,
provided that R is other than an unsubstituted morpholine group.

2. A compound according to claim 1 wherein R is a substituted morpholino, or a substituted or unsubstituted thiomorpholino group.

3. A compound according to claim 1 or 2 wherein n is 2.

4. A compound according to any one of claims 1 to 3 wherein R is unsubstituted or substituted with two methyl groups.

5. A compound according to any one of claims 1 to 4 and selected from :
6-amino-9-[(2-hydroxy-3-thiomorpholino)propyl]purine;
6-amino-9-([2-hydroxy-3-(2,6-cis-dimethylmorpholino)]

propyl)purine;
6-amino-3-[(2-hydroxy-3-thiomorpholino)propyl]purine, and
6-amino-3-([2-hydroxy-3-(2,6-cis-dimethylmorpholino)]propyl)-purine.

6. A process for producing a compound of the formula (I) or (II) according to claim 1, which process comprises:

(a) reacting adenine or a metal salt thereof with a compound of theformula (VIII):

$$Z - CH_2 - CHA - CH_2 - R \qquad (VIII)$$

wherein R is as defined in formulae (I) and (II); and A is hydroxyl and Z is a group readily displaceable by a nucleophile; or A and Z together form an oxide diradical; or

(b) the reaction of a compound of the formula (IX) or (X):

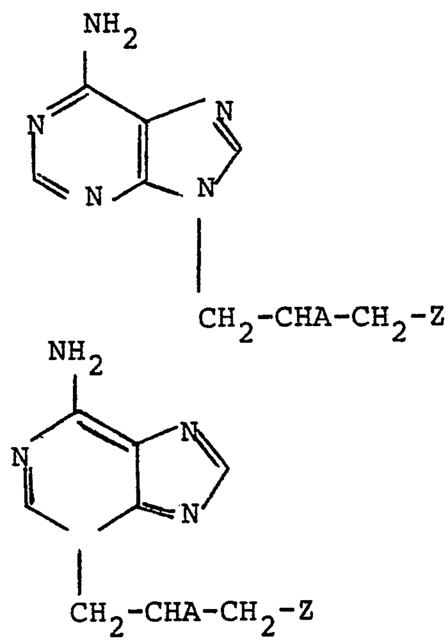

(IX)

(X)

wherein: A and Z are as hereinbefore defined, with a compound of the formula (XI):

$$H - R \qquad (XI)$$

wherein: R is as defined in formulae (I) and (II); or

c. the reduction of a compound of the formula (XII) or (XIII):

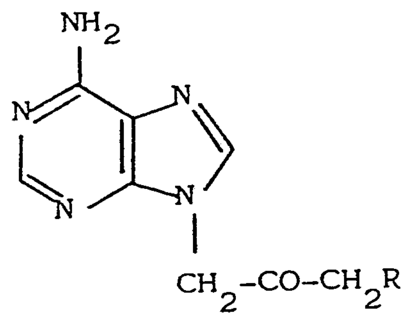

(XII)

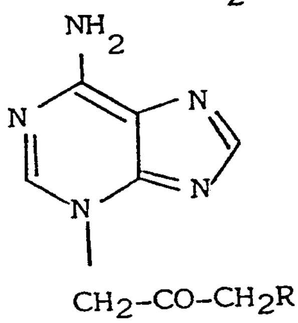

(XIII)

wherein R is as defined in formulae (I) and (II); or

d. the deprotection of an N- or O- protected compound corresponding to a compound of the formula (I) or (II); or

e. for a compound of the formula (I), the reaction of formamide with a compound of the formula (XIV):

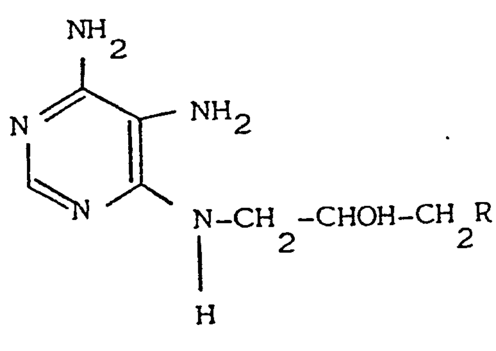

(XIV)

wherein R is as defined in formulae (I) and (II); and optionally thereafter salifying the compound of the formula (I) and (II) so produced.

7. A process as claimed in claim 6 (a) wherein Z is a bromine, chlorine, methanesulphonyloxy or toluenesulphonyloxy.

8. A process according to claim 6 (d) which comprises removing the oxide radical from a compound of the formula (XV):

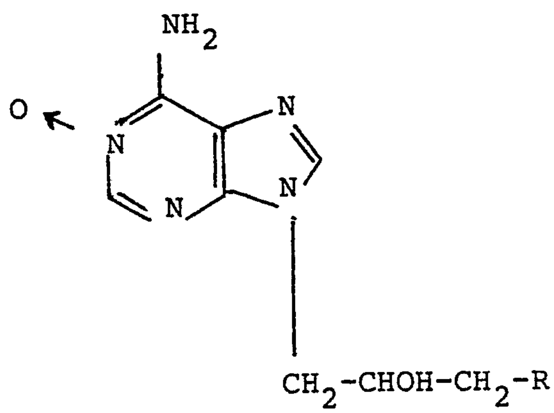

(XV)

9. A pharmaceutical composition which comprises a compound of formula (I) or (II) as claimed in claim 1 together with a pharmaceutically acceptable carrier therefor.

10. A composition as claimed in claim 9 provided in unit dosage form containing from 5 to 500 mgs of the compound of formula (I) or (II).

0052964

European Patent Office

# EUROPEAN SEARCH REPORT

Application number
EP 81 30 5218

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | JOURNAL OF MEDICINAL CHEMISTRY, vol. 11, no. 6, 1968, Washington (US) C. TEMPLE et al.: "Synthesis of Potential Antimalarial Agents. I.1 6-and 6,9-Disubstituted Purines" pages 1213-1215 | | C 07 D 473/34<br>A 61 K 31/52 |
| | * Page 1213, table I, compound 39 * | 1,9-10 | |
| | -- | | |
| Y | GB - A - 1 530 166 (J.A. WULFING) | | |
| | * Complete patent * | 1,6,9-10 | |
| | -- | | |
| P,A | EP - A - 0 024 122 (J.A. WULFING) | | |
| | ---- | | |

TECHNICAL FIELDS SEARCHED (Int.Cl. 3)

C 07 D 473/00
A 61 K 31/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 18-02-1982 | NUYTS |

EPO Form 1503.1 06.78